# EUROPEAN PATENT APPLICATION

(11) **EP 2 402 304 A1**
(43) Date of publication of application: **04.01.2012**
(21) Application number: 11170643.8
(22) Date of filing: 21.06.2011
(51) Int. Cl.: C07C 45/73, C07C 49/04

(54) **Method for preparing a ketone**

(30) Priority: 30.06.2010 US 360249 P
(71) Applicant: Rohm and Haas Company, Philadelphia, Pennsylvania 19106-2399 (US)
(72) Inventor: Martenak, Daniel, Perkasie, PA Pennsylvania 18944 (US); Tate, James F., New Castle, DE Delaware 19720 (US); Trejo-O' Reilly, Jose Antonio, Lansdale, PA Pennsylvania 19446 (US)
(74) Representative: Buckley, Guy Julian

(57) **Abstract**

A method for preparing a ketone, and ketone produced therefrom, comprising charging to a column a catalyst of an ion exchange resin impregnated with a metal chelate, adding solvent to the column, and initiating production of the ketone by flowing the solvent and hydrogen through the column.

## Description

This invention relates to methods preparing ketones. More particularly, this invention relates to methods for using complexed ion exchange resins to prepare ketones.

Methods for preparing ketones are known. U.S. Patent No. 6,977,314 discloses an acid-catalyzed condensation reaction using a metal-doped polysulfonated ion exchange resin catalyst. Before the reaction, the catalyst is reduced so that the metal is in elemental form.

GB1191113 also discloses the preparation of methyl isobutyl ketone by passing acetone and hydrogen downwardly over a fixed bed of strongly acidic catalyst containing Pd, Ru or Rh in divided form, the Pd, Ru or Rh having been introduced into the catalyst by impregnation with a salt solution of the metal and subsequent reduction of salt to metal at elevated temperature using hydrogen. Both of these methods require an extra step of reduction to use the catalyst.

The invention provides a method for preparing a ketone without the extra step of activation of the catalyst with reducing agents. This method allows for greater production efficiency after the catalyst is charged in the column in ketone synthesis and generates less waste in the form of undesirable products.

In a first aspect of the invention, there is provided a method for preparing a ketone comprising charging to a column a catalyst of an ion exchange resin impregnated with a metal chelate, adding solvent to the column, and initiating production of the ketone by flowing the solvent and hydrogen through the column.

In a second aspect of the invention, there is provided a method of making a ketone comprising charging to a column a catalyst of an ion exchange resin impregnated with 0.1 to 15 % metal chelate, based on dry weight of the catalyst, the metal chelate having a metal ion selected from at least one of palladium, platinum, indium, rhodium, ruthenium, copper, gold, and silver, adding solvent to the column, initiating production of the ketone by flowing the solvent and hydrogen through the column, and converting the solvent to ketone at a conversion rate of at least 5%.

In a third aspect of the invention, there is provided a ketone made by the method of the invention.

The invention is directed to a method of preparing a ketone. A fixed-bed reactor, or column, is charged with a metal complexed ion exchange resin catalyst. This catalyst comprises an ion exchange resin impregnated with a metal chelate. The metal chelate is not reduced. For example, if the metal is palladium, Pd(II) is the metal chelate. Pd(0) would be the reduced metal.

Examples of ion exchange resins include undersulfonated resins and polysulfonated resins. In a preferred embodiment, the ion exchange resin comprises a polysulfonated cation exchange resin, where the range of aromatic/sulfonic is from 10:1 1 to 1:2. The 1:2 is the sulfonation limit. Other resins that may be used for catalysis include acrylic backbone resins, such as weak acid cation resins, weak base anion resins, strong base anion resins and strong acid cation resins.

The ion exchange resins useful in the method may be in the form of a gel or macroporous beads. Preferably, the ion exchange resin catalysts are in the form of macroporous spherical beads having average particle diameters from 100 µm to 2 mm, more preferably, from 150 µm to 1.5 mm, and most preferably, from 250 to µm to 1 mm. When the ion exchange resin is a polysulfonated cation exchange resin, the content of the sulfonic acid group comprises, preferably, about 5.0 to 7.0, more preferably, about 5.1 to 6.5, and most preferably, about 5.2 to 6.0 meq/g (milliequivalents/gram), based on the dry weight of the polysulfonated cation exchange resin and is loaded with, preferably, about 0.1 to 10%, more preferably, about 0.5 to 5%, and most preferably, about 0.7 to 2%, of metal or metal ion, based on the dry weight of polysulfonated cation exchange resin.

Preferably, the ion exchange resin possesses a surface area from about 10 to 1000, more preferably, about 15 to 500, and most preferably, about 0.1 to 50 square meters/gram (m²/g) and, preferably, has a total porosity of about 0.1 to 0.9, more preferably, about 0.2 to 0.7, and most preferably, about 0.25 to 0.5 cubic centimeter pores per gram of polymer (cm³/g), with an average pore diameter of, preferably, about 50 to 2,500 Angstroms and more preferably, about 150 to 1000 Angstroms.

The ion exchange resins may be prepared from crosslinked macroporous copolymers, which are polymers or copolymers polymerized from a monomer or mixture of monomers containing at least 1 weight percent, based on the total monomer weight, of polyvinyl unsaturated monomer. The porosity is introduced into the copolymer beads by suspension-polymerization in the presence of a porogen (also known as a "phase extender" or "precipitant"), that is, a solvent for the monomer, but a non-solvent for the polymer.

A crosslinked macroporous copolymer preparation, for example, may include preparation of a continuous aqueous phase solution containing suspension aids (such as dispersants, protective colloids and buffers) followed by mixing with a monomer mixture containing 1 to 85% polyvinylaromatic monomer, free-radical initiator, and, preferably, about 0.2 to 5, more preferably, about 0.3 to 3, and most preferably, about 0.4 to 1, parts porogen (such as toluene, xylenes, (C₄ -C₁₀)-alkanols, (C₆ -C₁₂)-saturated hydrocarbons or polyalkylene glycols) per one part monomer. The mixture of monomers and porogen is then polymerized at an elevated temperature and the porogen is subsequently removed from the resulting polymer beads by various means, for example, toluene, xylene and (C₄ - C₁₀)alcohols may be removed by distillation or solvent washing and polyalkylene glycols may be removed by water washing. The resulting macroporous copolymer is then isolated by conventional means, such as dewatering followed by drying.

Suitable polyvinylaromatic monomers that may be used in the preparation of the crosslinked copolymers include, for example, one or more monomers selected from divinylbenzene, trivinylbenzene, divinyltoluene, divinylnaphthalene and divinylxylene, and mixtures thereof; it is understood that any of the various positional isomers of each of the aforementioned crosslinkers is suitable. In a preferred embodiment, the polyvinylaromatic monomer is divinylbenzene. Preferably, the crosslinked copolymer comprises about 1 to 85%, more preferably, about 5 to 55%, and most preferably, about 10 to 25%, polyvinylaromatic monomer units.

Optionally, non-aromatic crosslinking monomers, such as ethyleneglycol diacrylate, ethyleneglycol dimethacrylate, trimethylolpropane triacrylate, trimethylolpropane trimethacrylate, diethyleneglycol divinyl ether, and trivinylcyclohexane, may be used in addition to the polyvinylaromatic crosslinker. When used, the non-aromatic crosslinking monomers preferably comprise as polymerized units, from about 0 to 10%, more preferably, about 0 to 5%, and most preferably, about 0 to 2% of the macroporous polymer, based on the total monomer weight used to form the macroporous copolymer.

Suitable monounsaturated vinylaromatic monomers that may be used in the preparation of crosslinked copolymers include, for example, styrene, α-methylstyrene, (C₁ - C₄)alkyl-substituted styrenes, halo-substituted styrenes (such as dibromostyrene and tribromostyrene), vinylnaphthalene, and vinylanthracene. Preferably, the monounsaturated vinylaromatic monomer is selected from styrene, (C₁ -C₄)alkyl-substituted styrenes, and mixtures thereof. Included among the suitable (C₁ -C₄)alkyl-substituted styrenes are, for example, ethylvinylbenzenes, vinyltoluenes, diethylstyrenes, ethylmethylstyrenes, and dimethylstyrenes. It is understood that any of the various positional isomers of each of the aforementioned vinylaromatic monomers is suitable. Preferably, the copolymer comprises about 15 to 99%, and more preferably, about 75 to 90%, monounsaturated vinylaromatic monomer units.

Optionally, non-aromatic monounsaturated vinyl monomers, such as aliphatic unsaturated monomers, for example, vinyl chloride, acrylonitrile, (meth)acrylic acids, and alkyl (meth)acrylates, may be used in addition to the vinylaromatic monomer. When used, the non-aromatic monounsaturated vinyl monomers may comprise as polymerized units, preferably, from about 0 to 10%, more preferably, from about 0 to 5%, and most preferably, from about 0 to 2% of the macroporous copolymer, based on the total monomer weight used to form the macroporous copolymer.

Porogens useful for preparing macroporous copolymers include hydrophobic porogens, such as (C₇ -C₁₀)aromatic hydrocarbons and (C₆ -C₁₂)saturated hydrocarbons, and hydrophilic porogens, such as (C₄ -C₁₀)alkanols and polyalkylene glycols. Suitable (C₇ - C₁₀)aromatic hydrocarbons include, for example, one or more of toluene, ethylbenzene, ortho-xylene, meta-xylene and para-xylene; it is understood that any of the various positional isomers of each of the aforementioned hydrocarbons is suitable. Preferably, the aromatic hydrocarbon is toluene or xylene or a mixture of xylenes or a mixture of toluene and xylene. Suitable (C₆ -C₁₂)saturated hydrocarbons include, for example, one or more of hexane, heptane and isooctane; preferably, the saturated hydrocarbon is isooctane. Suitable (C₄ -C₁₀)alkanols include, for example, one or more of isobutyl alcohol, tert-amyl alcohol, n-amyl alcohol, isoamyl alcohol, methyl isobutyl carbinol (4-methyl-2-pentanol), hexanols and octanols; preferably, the alkanol is selected from one or more (C₅ -C₈)alkanols, such as, methyl isobutyl carbinol and octanol.

Polymerization initiators useful in preparing copolymers include monomer-soluble initiators, such as peroxides, hydroperoxides and related initiators, for example benzoyl peroxide, tert-butyl hydroperoxide, cumene peroxide, tetralin peroxide, acetyl peroxide, caproyl peroxide, tert-butyl peroctoate (also known as tert-butylperoxy-2-ethylhexanoate), tert-amyl peroctoate, tert-butyl perbenzoate, tert-butyl diperphthalate, dicyclohexyl peroxydicarbonate, di(4-tert-butylcyclohexyl)peroxydicarbonate, and methyl ethyl ketone peroxide. Also useful are azo initiators, such as azodiisobutyronitrile, azodiisobutyramide, 2,2'-azo-bis(2,4-dimethylvaleronitrile), azo-bis(.α-methylbutyronitrile) and dimethyl-, diethyl- or dibutyl azo-bis(methylvalerate), Preferred peroxide initiators are diacyl peroxides, such as benzoyl peroxide, and peroxyesters, such as tert-butyl peroctoate and tert-butyl perbenzoate; more preferably, the initiator is benzoyl peroxide. Use levels of peroxide initiator are, preferably, about 0.3% to 5%, more preferably, about 0.5 to 3%, and most preferably, about 0.7 to 2%, based on the total weight of vinyl monomers.

Preferably, the crosslinked copolymers are selected from divinylbenzene copolymer, styrene-divinylbenzene copolymer, divinylbenzene-ethylvinylbenzene copolymer and styrene-ethylvinylbenzene-divinylbenzene copolymer for use as substrates for the catalysts. These crosslinked copolymers may be functionalized with strong-acid functional groups according to conventional processes for polysulfonation known to those having ordinary skill in the art, as for example, sulfonation with sulfur trioxide (SO₃), fuming sulfuric acid or oleum (concentrated sulfuric acid containing sulfur trioxide), and chlorosulfonic acid. Alternatively, monosulfonated cation exchange resin polymers may also be subjected to conventional polysulfonation conditions to provide the polysulfonated cation exchange resin catalysts.

The catalyst also comprises a metal chelate. Exemplary metal chelates, or metal ions, include palladium (Pd(II)), platinum (Pt(II)), iridium (Ir(III)), rhodium (Rh(III)), ruthenium (Ru(III)), copper (Cu(I)), gold (Au(I)), silver (Ag(I)), and mixtures thereof.

The ion exchange resins may be loaded with the desired metal ion by contacting an aqueous solution of the metal ion with the hydrogen form of the ion exchange resin in a batch or continuous reactor. The metal ion may be provided in the form a metal salt, such as, for example, chlorides, bromides, nitrates, sulphates, acetylacetonates, and acetates. The loaded ion exchange resin may be rinsed free of residual salts or acid. The amount of metal salt used is chosen such that the metal or metal ion will ultimately be present in an amount of about 0.1 to 2% loading, preferably about 0.5 to 1.5% loading, and more preferably about 0.8 to 1.2% loading of ion exchange resin. Preferably, the ion exchange resin catalysts contain 0.1 to 15% metal, based on dry weight of the catalyst.

The packing of the catalyst is improved when the column is packed with the use of a solvent other than water. The solvent is preferably acetone. Other solvents and products include, but are not limited to methyl isobutyl ketone, isopropanol, isobutanol, methylisobutylcarbinol, methanol, toluene, tetrahydrofurane, and dioxane.

In a preferred embodiment of the invention, the metal complexed ion exchange resin catalyst is in the physical form of beads contained in a vessel, the beads forming a bed of the catalyst. A feed stream of ketone reactant, or solvent, such as acetone, is brought into contact with the catalyst bed in the presence of hydrogen (as a separate feed stream) for a sufficient time and temperature for a condensation reaction of the ketone to occur. The condensed liquid stream, containing reaction products (saturated ketone adduct), byproducts (unsaturated ketone adduct), and any unreacted ketone reactant that may be present, is separated from the catalyst bed, and desired ketone adduct is recovered from the liquid stream by conventional separation means (such as distillation).

One of ordinary skill in the art will be able to choose appropriate conditions, such as (1) batch operation, for example, in which the catalyst bed is loaded with the liquid stream in the presence of hydrogen, or (2) the more preferred continuous operation, for example, where the liquid stream is fed continuously into one end of a column reactor (with hydrogen) at a rate that allows sufficient residence time in the catalyst bed for the desired reaction to occur, with the condensed liquid stream being removed continuously from the other end of the bed Similarly, the reaction equipment, the choice of upflow or downflow for the direction of passage of the reactant streams through the bed, the reaction time and temperature, the particular reactants, and the method of recovering the ketone adduct, are readily selected based upon the guidance provided herein and the knowledge available to one of ordinary skill in the art.

The temperatures and pressures inside the column reactor may be selected so that the ketone reactant is at its boiling point in the catalyst bed. Variation of temperature/pressure of the ketone reactant is used to provide the desired combination of reaction temperature and conditions such that the condensation reaction takes place in the liquid phase in the catalyst bed. Conditions may be varied to provide gas phase conditions with the catalyst bed, and the conditions may be such that the condensation reaction is conducted in the liquid phase. In a preferred embodiment, a trickle bed condition, where there is liquid and gas flowing through the catalyst bed, is used. In one embodiment, the gas is hydrogen and the equilibrium liquid/vapor is acetone. Choosing a higher pressure may provide more liquid.

The solvent and hydrogen may be contacted under batch reaction conditions or under continuous reaction conditions. In one embodiment, the method is a continuous process based on a catalytic distillation process with the introduction of the ketone reactant being into the bottom of a column reactor immediately above a reboiler stage; in this case, the product fraction or stream is withdrawn continuously from the reboiler portion of the distillation apparatus for further processing. Preferably, the ketone reactant to undergo the condensation reaction is fed downward through the catalyst bed and a current of hydrogen is passed through the reaction zone in the same direction. However, other variations of introducing the reactant feed streams may be used, such as co-current and countercurrent hydrogen flow, flooding processes, and gaseous-phase processes.

For continuous processes, the amount of catalyst to be used, relative to the amount of reactants, is typically related to the throughout rate of the reactions, as indicated by the LHSV (liquid hourly space velocity) or liquid flow rate of reactants relative to the volume of catalyst per unit time. High LHSV may be desirable to maximize equipment usage and generation of product; however, meeting this objective must be balanced against % conversion of raw materials and % selectivity to the desired product. If the LHSV is too low, production rate of the desired product (yield and selectivity) is diminished, and the process may not be economical. If the LHSV is too high, the catalyst activity will be insufficient to provide the desired level of conversion (the process becomes "kinetically limited"). Suitable values of LHSV will typically range from, preferably, 0.5 and 10 h⁻¹, more preferably, from 1 to 8 h⁻¹, and most preferably, from 2 to 4 h⁻¹.

The ketone reactact, or solvent, may be contacted with hydrogen in the presence of the catalyst at a temperature of 65 to 200 °C and at a pressure from 1 to 100 bar (0.1 to 10 MPa) of hydrogen. Typically, the condensation reaction is conducted at a hydrogen/ketone reactant molar ratio of at least 1:1.

In another embodiment of the invention, the process may be a batch reaction with the introduction of the solvent into a reactor column at the reboiler section stage of a catalytic distillation apparatus (similar to that described above). The process may then be terminated when a desired product composition of ketone adduct is achieved in the reboiler section. Alternatively, the condensation may be carried out in a batch autoclave reactor for a specified period of time, followed by cooling and recovery of the desired of the ketone adduct by distillation or other conventional means.

The ketone is prepared by converting the solvent to a desired ketone product at a preferred conversion rate of at least 5%. A more preferred conversion rate is 5-65% and a most preferred conversion rate is 20-38%. The ketone product may be a ketone or product with ketone functionality. The yield comprises about 5-65% and a selectivity of about 70-99%. Yield is based on the amount of ketone produced and selectivity is based on the amount of ketone produced relative to the total product.

The following examples are presented to illustrate the invention. In the examples, the following abbreviations have been used.
atm is atmospheres.
%-w is percent by weight.
GC is gas chromatograph.
kPa is kilopascal.
LHSV is liquid hourly space velocity.
MIBK is methyl isobutyl ketone.
MPa is megaPascal.
psi is pounds per square inch.
C is Celsius; ml is milliliter; µl is microliter; s is second; min is minute; h is hour; m is meter; cm is centimeter; mm is millimeter; and nml/min is milliliter per minute at gas standard conditions defined as pressure = 1 atm, temperature = 25 °C, and volume = 22.4 liters.

### TEST METHODS

Yield, Conversion, and Selectivity: The product from reaction is injected in a GC chromatograph. The different reaction products were analyzed and quantified. The acetone conversion is the acetone that reacts to make products, the product yield is the amount of wanted product obtained, and the selectivity is the ratio of target product to all the products determined by GC.

Dual column GC-FID Method description:
Carrier Gas: N₂ from High Pressure house Nitrogen
Injector: 0.2 µl volume
Inlet: Front, Mode: split, Temperature: 250°C, Pressure: 5.4 psi (37 kPa)
Split ratio: 50.0 to 1, Split flow 73.0 ml/min; Total flow 76.6 ml/min
Gas saver: 20.0 ml/min @ 2.00 min

Columns:
Column 1: Macherei Nagel 726600. Optima Wax. 30m x 250 µm x 0.25 µm
Constant Pressurure, Inlet: Front, Outlet: Front
Nitrogen flow: Pressure 5.4 psi (37 kPa), Flow 0.7 ml/min, Average velocity 20 cm/s
Column 2: Varian CP9151 VF1701MS Capillary 30.0 m x 250 µm x 0.25 µm
Constant Pressure, Inlet: Front, Outlet: Back
Nitrogen flow: Pressure 5.4 psi (37kPa), Flow 0.7 ml/min, Average velocity 20 cm/s

Oven:
Setpoint: 40°C
Hold time: 5 min
Ramp 1: 5.0°C/min to 115°C
Ramp 2: 15.0°C/min to 240°C
Final time: 6.67 min @ 240°C
Total run time: 35 min

Detectors:
Front FID: Heater: 250°C
Flows: H₂: 30 ml/min, Air: 350 ml/min, Makeup N₂; 30 ml/min
Signal 1: Data rate 20 Hz, peak width 0.01 min, Start 0, End 35 min
Back FID: Heater: 250°C
Flows: H₂: 30 ml/min, Air: 350 ml/min, Makeup N₂: 30 ml/min
Signal 2: Data rate 20 Hz, peak width 0.01 min, Start 0, End 35 min

**Table 1: Standards for Testing for Yield, Conversion, and Selectivity**

| Compound Name | CAS # |
|---|---|
| Acetone | |
| Benzene, 1,2,4 trimethyl- | 95-63-6 |
| Diacetone alcohol | 123-42-2 |
| Diisobutyl ketone (DMH1) | 108-83-8 |
| 2-Heptanone, 4,6-dimethyl-(DMH2) | 19549-80-5 |
| Isopropyl alcohol | 67-63-0 |
| 4-Methyl-2-pentanol (MIBC) | 108-11-2 |
| Methyl Isobutyl Ketone (MIBK) | 108-10-1 |
| 3-Penten-2-one, 4-methyl- (MSO) | 141-79-7 |
| Pentane, 2-methyl- | 107-83-5 |

### EXAMPLES

### Example 1 Comparative Example of MIBK Synthesis

15 ml of AMBERLYST™CH28 resin with Pd(II) was used for a packed bed. The resin is slurry in water and the catalyst bed is packed. Hydrogen was flowed through the column at 100°C for 48 hours and the Pd(II)was reduced to Pd(0). Dehydration of the system was required and water replaced by acetone in the column. After this exchange was finished, the system was then ready to start MIBK production. The reactor temperature was increased to the reaction set point and hydrogen flow rate of 250 ml/min at a pressure of 20 atm (2.03 MPa). The acetone flow rate chosen for this reaction was 1 ml/min (LHSV 1 (h⁻¹)). The product obtained after 3 hours was collected and analyzed in a GC equipment. A next temperature was then used and after 3 hours, samples were taken. Several temperatures were used: 90°C, 100°C, 110°C, 120°C, and 130°C. Acetone conversion and selectivity are shown in Table 1.

### Example 2 MIBK Synthesis

15 ml of AMBERLYST™CH28 resin with Pd(II) was used for a packed bed. The resin was slurry in acetone prior to packing the column. The column was packed with acetone. The reactor temperature was increased to the reaction set point (Temperature = 100°C and 120°C) and hydrogen flow rate of 250 ml/min at a pressure of 20 atm (2.03 MPa). The acetone flow rate chosen for this reaction was 1 ml/min (LHSV 1 (h⁻¹)). The product obtained after 3 hours was collected and analyzed in a GC equipment for each temperature. Acetone conversion and selectivity are shown in Table 1.

**Table 1: Acetone Conversion and Selectivity**

| | Example 1 | Example 2 |
|---|---|---|
| Pd valence | Pd(0) | Pd(II) |
| Acetone Conversion (%-w) | 48 | 55 |
| Carbon Accountability (%-w) | 98 | 98 |
| MIBK Yield (%-w) | 44 | 48 |
| MIBK Selectivity (%) | 92 | 92 |
| Reaction Temperature (°C) | 120 | 120 |
| Hydrogen Flowrate (nml/min) | 300 | 300 |
| Acetone LHSV (h⁻¹) | 2 | 2 |
| Pressure (MPa) | 2.7 | 2.7 |

Higher acetone conversion was obtained when Pd(II) catalyst was used.

## Claims

1. A method for preparing a ketone comprising:
charging to a column a catalyst of an ion exchange resin impregnated with a metal chelate;
adding solvent to the column; and
initiating production of the ketone by flowing the solvent and hydrogen through the column.

2. The method of claim 1 wherein the solvent comprises acetone.

3. The method of claim 1 wherein the metal chelate comprises a metal ion selected from at least one of palladium, platinum, iridium, rhodium, ruthenium, copper, gold, and silver.

4. The method of claim 1 wherein the metal chelate comprises Pd(II).

5. The method of claim 1 wherein the catalyst comprises 0.1 to 15 % metal chelate, based on dry weight of the catalyst, distributed therein,.

6. The method of claim 1 further comprising a yield of 5-65% and a selectivity of 70-99%.

7. The method of claim 1 wherein the ketone comprises methyl isobutyl ketone.

8. The method of claim 1 further comprising:
converting the solvent to ketone at a conversion rate of at least 5%.

9. A ketone made by the method of claim 1.

10. A method of making a ketone comprising:
charging to a column a catalyst of an ion exchange resin impregnated with 0.1 to 15 % metal chelate, based on dry weight of the catalyst, the metal chelate having a metal ion selected from at least one of palladium, platinum, iridium, rhodium, ruthenium, copper, gold, and silver;
adding solvent to the column;
initiating production of the ketone by flowing the solvent and hydrogen through the column; and
converting the solvent to ketone at a conversion rate of at least 5%.
